# EUROPEAN PATENT APPLICATION

(11) **EP 1 840 227 A1**
(43) Date of publication of application: **03.10.2007**
(21) Application number: 07250877.3
(22) Date of filing: 02.03.2007
(51) Int. Cl.: C12Q 1/68

(54) **Markers for melanoma**

(30) Priority: 02.03.2006 WO PCT/GB2006/000756; 03.03.2006 GB 0604370
(71) Applicant: UNIVERSITY COLLEGE DUBLIN, Dublin 4 (IE)
(72) Inventor: Gallagher, William, Dublin 4 (IE); Rafferty, Mairim, Dublin 4 (IE); Faller, William, Dublin 4 (IE)
(74) Representative: Spencer, Matthew Peter

(57) **Abstract**

A method of diagnosing melanoma and/or monitoring melanoma progression, in particular for determining whether the melanoma is likely to have metastatic capabilities, in a subject includes the steps of in a test sample determining the methylation status of FABP5 wherein methylation of the gene indicates a positive diagnosis of melanoma and/or increased methylation of the gene indicates the progression of melanoma. Methods of monitoring melanoma progesssion may also incorporate measuring the expression levels of FABP5, with low levels of expression indicating a positive diagnosis of melanoma and/or lower levels of expression being indicative of progression of melanoma. Methods of treating melanoma in a subject comprise administering a therapeutically effective amount of a DNA methyltransferase inhibitor or a histone deacetylase inhibitor to the subject such that expression of FABP5 is increased. Microarrays for use in these diagnostic methods and compound screening methods based around monitoring methylation and/or expression of FABP5 are also envisaged.

## Description

### TECHNICAL FIELD

The present invention relates to methods and products useful for diagnosing melanoma and is based around the unexpected finding of new markers associated with melanoma and its progression.

### INTRODUCTION

The incidence of cutaneous melanoma is at epidemic proportions, with rates steadily rising in Western countries over the past few decades (1, 2). However, effective treatment for patients with advanced melanoma is currently unavailable. Moreover, the prognosis of such patients is poor, with a 10% survival rate after 5 years. Less than a decade ago, cutaneous melanoma was described as a "black tumor" and a "black box" (3). While considerable insights have recently been made with respect to mapping out central events in melanoma development, the molecular basis of tumor progression in this disease remains ill-defined. One approach towards understanding melanoma is to compare gene expression patterns between melanocytic cells from different stages of tumor progression. In this context, DNA microarray-based gene expression profiling has had a far-reaching impact on the study of numerous tumor types (4), including melanoma (5). Using this approach, new subgroups of melanoma have been identified (6), along with a wealth of marker genes that correlate with melanoma progression and drug response (7-13).

Cutaneous melanoma is a pigmented, readily accessible lesion that has been well-defined in histopathological terms (3). Early radial growth phase (RGP) melanomas can invade into the epidermis and papillary dermis, but have no capacity for metastasis; resection at this stage is almost completely curative. A subsequent vertical growth phase (VGP) denotes a transition to a more aggressive stage, which is capable of metastasis. Changes in gene expression occurring at the RGP/VGP transition are, thus, of great interest. However, comparative transcriptomic studies have so far been hindered in this arena, as paired RGP/VGP biopsies are not normally available (since resection of RGP melanoma is often curative and no VGP develops).

### DESCRIPTION OF THE INVENTION

The present invention relates to methods and products useful for diagnosing and monitoring the progession of melanoma and is based around the unexpected finding of a new marker associated with melanoma and its progression.

Accordingly, there is provided, in a first aspect of the invention, a method of diagnosing melanoma, in particular monitoring the progression of melanoma with respect to more invasive forms, in a subject comprising, in a test sample, determining expression levels of FABP5 wherein a statistically significant decrease in the expression of FABP5 indicates the presence of melanoma or progression of melanoma to a more invasive form in the subject.

Note that all genes are listed utilising standard nomenclature approved by the HUGO Gene Nomenclature Committee (HGNC) on behalf of the human genome organisation to ensure that each symbol is unique. Accession numbers providing full sequence information and further details can be found at the HGNC website (www.gene.ucl.ac.uk/nomenclature). Unigene accession numbers for the FABP5 gene are as follows:
FABP5 - Bt22869, SSc.5549, Rn.98269, Mm741, Hs408061, Gga3323. Chromosome location 8q21.12, Seq acc no: M94856.

Of course, generally human samples will be assessed for the expression level or methylation of the human FABP5 gene. However, suitable homologues or orthologues may be investigated as appropriate in other contexts (such as in model organisms).

The method is most preferably an in vitro method carried out on an isolated sample. In one embodiment the method may also include the step of obtaining the sample.

The method may preferably be utilised to determine if a potential melanoma is in early radial growth phase (RGP) or a subsequent vertical growth phase (VGP). Thus, the methods of the invention are capable of determining the aggressiveness of a melanoma, in particular whether VGP has been reached which may lead to different treatment regimes being selected.

Prior to the present invention, it was not known that the expression of this gene is linked to the incidence and progression of melanoma and is down-regulated as melanoma progresses to more aggressive forms, which are eventually capable of metastasis. Generally, the method may be utilised in order to monitor progression of a melanoma to ensure it does not progress to a potentially metastatic lesion and to allow suitable treatment if melanoma, in particular metastatic melanoma, is suspected.

The test sample is most preferably a tissue sample, taken from the subject, which is suspected of being tumorigenic. In a most preferred embodiment, the sample comprises melanocytes suspected of being a melanoma. However, any other suitable test sample in which expression of the novel marker of the present invention can be measured to indicate the presence of a melanoma in particular a melanoma in VGP, are included within the scope of the invention. Test samples for diagnostic, prognostic, or personalized medicine uses can be obtained from surgical samples, such as biopsies or fine needle aspirates, from paraffin embedded tissues, or from a body fluid.

The increased or decreased level of expression must be statistically significant in order to provide a reliable test for monitoring melanoma. Any method for determining whether the expression level of the gene is significantly increased or decreased may be utilised. Such methods are well known in the art and routinely employed. For example, statistical analyses may be performed using an analysis of variance test. A typical P value for use in such a method would be P values of < 0.05 when determining whether the relative expression is statistically significant. A change in expression may be deemed significant if there is at least a 10% increase or decrease for example. The test may be made more selective by making the change at least 15%, 20%, 25%, 30%, 35%, 40% or 50%, for example, in order to be considered statistically significant.

In a preferred embodiment, the increased or decreased level of expression is determined with reference to a control sample. This control sample is preferably taken from normal (i.e. non malignant) melanocytes in the subject.

Alternatively, the control sample is taken from the same tissue as that under test at an earlier time point. This is particularly relevant for monitoring progression of a melanoma, for example in order to ensure that treatment has been effective to prevent progression to an aggressive form of the melanoma. Thus, the progression, or effective prevention of progression, of a potential melanoma for example from radial to vertical growth phase can be monitored.

Suitable additional controls may also be included to ensure that the test is working properly, such as measuring expression of a suitable reference gene in both test and control samples.

In a most preferred embodiment, the subject is a human subject. Generally the subject will be a patient wherein a potential melanoma has been identified and the method may be used to determine if indeed there is a potentially dangerous lesion and also to guide treatment depending upon the stage of progression of the melanoma.

It is believed that the FABP5 marker is particularly useful in the diagnosis, prognosis and for monitoring the progression, of melanoma.

Bisulphite sequencing has revealed that the promoter region of the FABP5 gene becomes progressively more methylated in more aggressive melanoma cell lines. Thus, changes in expression associated with methylation reflect the progression of melanoma to more aggressive forms.

Expression of a gene can be assessed using any means known in the art. Either mRNA or protein can be measured.
Methods employing hybridization to nucleic acid probes can be employed for measuring specific mRNAs. Such methods include using nucleic acid probe arrays (microarray technology) and using Northern blots. Messenger RNA can also be assessed using amplification techniques, such as RT-PCR. Advances in genomic technologies now permit the simultaneous analysis of thousands of genes, although many are based on the same concept of specific probe-target hybridization. Sequencing-based methods are an alternative; these methods started with the use of expressed sequence tags (ESTs), and now include methods based on short tags, such as serial analysis of gene expression (SAGE) and massively parallel signature sequencing (MPSS).
Differential display techniques provide yet another means of analyzing gene expression; this family of techniques is based on random amplification of cDNA fragments generated by restriction digestion, and bands that differ between two tissues identify cDNAs of interest. Specific proteins can be assessed using any convenient method including immunoassays and immuno-cytochemistry but are not limited to these specific methods. Most such methods will employ antibodies which are specific for the particular protein or protein fragments.

In one embodiment, the levels of gene expression are determined using RT-PCR. Reverse transcriptase polymerase chain reaction is a well known technique in the art which relies upon the enzyme reverse transcriptase to reverse transcribe mRNA to form cDNA, which can then be amplified in a standard PCR reaction. Protocols and kits for carrying out RT-PCR are extremely well known to those of skill in the art and are commercially available.

Suitable primers for use in the methods of the invention include:
FABP5-For: TCA GCA GCT GGA AGG AAG AT (SEQ ID NO: 8)
FABP5-Rev: GCC ATC AGC TGT GGT TTC TT (SEQ ID NO: 9)

In a preferred embodiment, the RT-PCR is carried out in real time and in a quantitative manner. Real time quantitative RT-PCR has been thoroughly described in the literature (see Gibson et al for an early example of the technique) and a variety of techniques are possible. Examples include use of Taqman, Molecular Beacons, LightCycler (Roche), Scorpion and Amplifluour systems. All of these systems are commercially available and well characterised, and may allow multiplexing (that is, the determination of expression of multiple genes in a single sample) which is particularly advantageous in the method of the present invention.

These techniques produce a fluorescent read-out that can be continuously monitored. Real-time techniques are advantageous because they keep the reaction in a "single tube". This means there is no need for downstream analysis in order to obtain results, leading to more rapidly obtained results. Furthermore keeping the reaction in a "single tube" environment reduces the risk of cross contamination and allows a quantitative output from the methods of the invention. This may be particularly important in the clinical setting of the present invention.

As an example, real-time quantification of PCR reactions can be accomplished using the TaqMan^{®} system (Applied Biosystems), see Holland et al; Detection of specific polymerase chain reaction product by utilising the 5'-3' exonuclease activity of *Thermus aquaticus* DNA polymerase; Proc. Natl. Acad. Sci. USA 88, 7276-7280 (1991) (32), Gelmini et al. Quantitative polymerase chain reaction-based homogeneous assay with flurogenic probes to measure C-Erbb-2 oncogene amplification. Clin. Chem. 43, 752-758 (1997)(33) and Livak et al. Towards fully automated genome wide polymorphism screening. Nat. Genet. 9, 341-342 (1995) (34), incorporated herein by reference. Taqman^{®} probes are widely commercially available, and the Taqman^{®} system (Applied Biosystems) is well known in the art. Taqman^{®} probes anneal between the upstream and downstream primer in a PCR reaction. They contain a 5'-fluorophore and a 3'-quencher. During amplification the 5'-3' exonuclease activity of the Taq polymerase cleaves the fluorophore off the probe. Since the fluorophore is no longer in close proximity to the quencher, the fluorophore will be allowed to fluoresce. The resulting fluorescence may be measured, and is in direct proportion to the amount of target sequence that is being amplified. Such probes are commonly referred to as hydrolytic probes.

In the Molecular Beacon system, see Tyagi & Kramer. Molecular beacons - probes that fluoresce upon hybridization. Nat. Biotechnol. 14, 303-308 (1996) (35) and Tyagi et al. Multicolor molecular beacons for allele discrimination. Nat. Biotechnol. 16, 49-53 (1998) (36) both of which are incorporated by reference herein, the beacons are hairpin-shaped probes with an internally quenched fluorophore whose fluorescence is restored when bound to its target. The loop portion acts as the probe while the stem is formed by complimentary "arm" sequences at the ends of the beacon. A fluorophore and quenching moiety are attached at opposite ends, the stem keeping each of the moieties in close proximity, causing the fluorophore to be quenched by energy transfer. When the beacon detects its target, it undergoes a conformational change forcing the stem apart, thus separating the fluorophore and quencher. This causes the energy transfer to be disrupted to restore fluorescence.

Any suitable fluorophore is included within the scope of the invention. Fluorophores that may possibly be used in the method of the invention include, by way of example, FAM, HEX^{™}, NED^{™}, ROX^{™}, Texas Red^{™} etc. Quenchers, for example Dabcyl and TAMRA are well known quencher molecules that may be used in the method of the invention. However, the invention is not limited to these specific examples. EDANS and DABCYL form a particularly efficient fluorophore/quencher pair (62), as do fluorescein/DABCYL (67).

A further real-time fluorescence based system which may be incorporated in the methods of the invention is Zeneca's Scorpion system, see Detection of PCR products using self-probing amplicons and fluorescence by Whitcombe et al. Nature Biotechnology 17, 804 - 807 (01 Aug 1999) (37). This reference is incorporated into the application in its entirety. The method is based on a primer with a tail attached to its 5' end by a linker that prevents copying of the 5' extension. The probe element is designed so that it hybridizes to its target only when the target site has been incorporated into the same molecule by extension of the tailed primer. This method produces a rapid and reliable signal, because probe-target binding is kinetically favoured over intrastrand secondary structures.

Amplifluour primers (as described in US Patent 5,866,336 to Nazarenko and WO98/02449 both of which are incorporated herein by reference) rely upon a similar principle to molecular beacons. However, in this case, the hairpin structure is part of the amplification primer itself. The primer binds to a nucleic acid strand and directs synthesis and thus becomes part of the amplification product. When the complementary strand is synthesised amplification occurs through the hairpin structure. This separates the fluorophore and quencher molecules, thus leading to generation of fluorescence as amplification proceeds.

It should be noted that whilst PCR is a preferred amplification method, equivalents may also be included within the scope of the invention. Examples include isothermal amplification techniques such as NASBA, 3SR, TMA and triamplification, all of which are well known in the art and commercially available (and are described in more detail below). Other suitable amplification methods include the ligase chain reaction (LCR) (Barringer et al, 1990), selective amplification of target polynucleotide sequences (US Patent No. 6,410,276), consensus sequence primed polymerase chain reaction (US Patent No 4,437,975), arbitrarily primed polymerase chain reaction (WO90/06995) and nick displacement amplification (W02004/067726).

In a further embodiment, the method of the invention incorporates use of microarrays in order to detect expression of the genes which represent novel melanoma markers. Suitable microarrays are described in further detail below.

In one embodiment, the method of the invention is used to determine whether the melanoma is likely to have metastatic capabilities. As shown in the experimental section below, the novel marker gene is significantly down-regulated in more aggressive forms of melanoma which may be capable of metastasis. Therefore, significantly decreased levels of expression may be indicative of a form of melanoma capable of metastasis (which may correspond to vertical growth phase).

As described in the experimental section below, bisulphite sequencing of the FABP5 promoter region reveals differential methylation. As melanoma progresses to more invasive or aggressive forms, the promoter region becomes increasingly methylated.

Accordingly, in a second aspect the invention provides a method of diagnosing melanoma in a subject comprising, in a test sample, determining the methylation status of FABP5 wherein increased methylation of the gene indicates the presence of melanoma. The method is one which allows melanoma progression to be monitored, in particular to determine whether the melanoma is likely to have metastatic capabilities.

The method is most preferably an *in vitro* method carried out on an isolated sample. In one embodiment, the method may also include the step of obtaining the sample.

The subject of the invention, as for the first aspect, is most preferably a human and the features described above in respect of that aspect of the invention also apply *mutatis mutandis* to this aspect of the invention.

As discussed above, the test sample is most preferably a tissue sample taken from the subject which is suspected of being a tumor. In a most preferred embodiment the sample comprises melanocytes suspected of being a melanoma. However, any other suitable test sample in which methylation status of the gene, which represents the novel marker of the present invention and markers with a known link to melanoma but without any knowledge of importance of methylation for melanoma progression, can be determined to indicate the presence of a melanoma are included within the scope of the invention.

Hypermethylation is a well known term in the art which denotes increased and aberrant methylation at specific CpG sites in a gene. Hypermethylation most often occurs in the promoter regions of genes and acts to depress expression of the gene. Accordingly, in a preferred embodiment, the method is carried out wherein the promoter regions of the relevant genes are assessed to determine their methylation status, in particular whether the promoter region is hypermethylated.

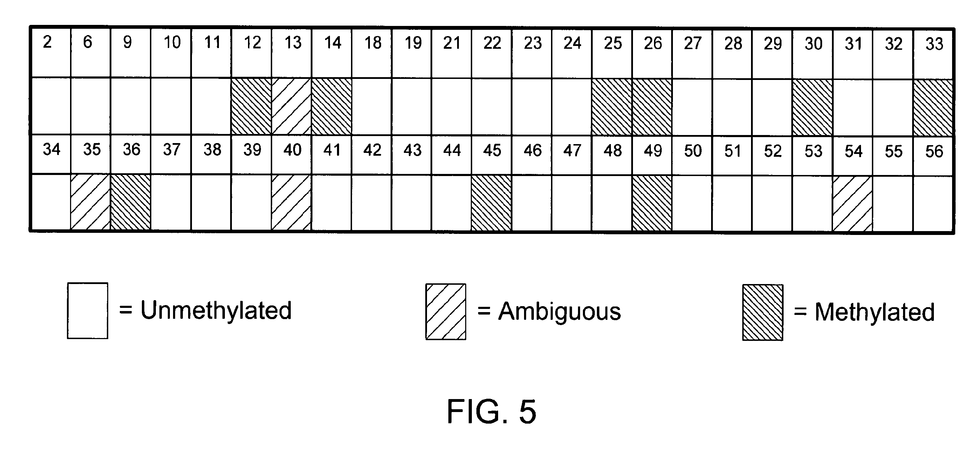

In a particularly preferred embodiment, the promoter region of the FABP5 gene is investigated for its methylation status. The promoter region and CpG island of the FABP5 gene are described in Figure 5 (SEQ ID NO: 1) and the CpG island is preferably investigated for determination of the methylation status.

Suitable primers for investigating the methylation status of the FABP5 gene using bisulphite sequencing include those comprising, consisting essentially of or consisting of the following nucleotide sequences:
Sense: 5'-TGG TTT TTT GGG TTT GTA GA (SEQ ID NO: 2)
Antisense: 5'-ATA CCT CAC CTA ACT CCT TCA T (SEQ ID NO: 3)

Suitable primers for investigating the methylation status of the FABP5 gene using methylation specific PCR include those comprising, consisting essentially of or consisting of the following nucleotide sequences:
Methylated, sense: TTT GGG TTT GTA GAG GCG TC (SEQ ID NO: 4)
, antisense TTA CCA AAA TAC GCG ACG AC (SEQ ID NO: 5)
Unmethylated, sense TTT TTT GGG TTT GTA GAG GTG TT (SEQ ID NO: 6)
, antisense ACC AAA ATA CAC AAC AAC CTC TC (SEQ ID NO: 7)

As is discussed above in respect of the first aspect of the invention, additional genes which represent novel methylation related melanoma markers can be assessed in order to provide a diagnosis. For example 1, 2, 3, 4, 5 or 6 etc. genes may be assessed in terms of their methylation status in the method of this aspect of the invention. In a most preferred embodiment, the methylation status of additionally at least TSPY1 is determined. In a further preferred embodiment, the methylation status of at least CYBA and/or MT2A (MT1E) is also determined. The methylation status of these genes is similarly thought to be linked to progression of melanoma, see International application publication number WO 2006/092610 to University College Dublin, filed 2 March 2006, which reference is incorporated herein in its entirety.

In a further preferred embodiment, the methylation status of the genes is determined using methylation specific PCR (MSP). However, any technique may be utilised to determine the methylation status of the genes, such as, for example, the well known techniques of COBRA (for more details reference is made to the experimental section below), bisulphite sequencing and use of arrays which can distinguish between bisulphite treated nucleic acid molecules following amplification. A review of some useful techniques is provided in Nucleic acids research, 1998, Vol. 26, No. 10, 2255-2264, which reference is incorporated herein in its entirety. DNA methylation analysis has been performed successfully with a number of techniques which include the MALDI-TOF, MassARRAY , MethyLight, Quantitative analysis of ethylated alleles (QAMA), enzymatic regional methylation assay (ERMA), HeavyMethyl, QBSUPT, MS-SNuPE, MethylQuant, Quantitative PCR sequencing, Oligonucleotide-based microarray systems. A further method for the identification of methylated CpG dinucleotides utilizes the ability of the MBD domain of the McCP2 protein to selectively bind to methylated DNA sequences (Cross et al, 1994; Shiraishi et al, 1999). Restriction enconuclease digested genomic DNA is loaded onto expressed His-tagged methyl-CpG binding domain that is immobilized to a solid matrix and used for preparative column chromatography to isolate highly methylated DNA sequences.

The MSP technique will be familiar to one of skill in the art. In the MSP approach, DNA may be amplified using primer pairs designed to distinguish methylated from unmethylated DNA by taking advantage of sequence differences as a result of sodium-bisulfite treatment (Herman et al (65) and see WO 97/46705, incorporated herein by reference). After sodium-bisulfite treatment unmethylated cytosines are converted to uracil whereas methylated cytosines remain unconverted.

A specific example of the MSP technique is designated real-time quantitative MSP (QMSP), which permits reliable quantification of methylated DNA in real time. These methods are generally based on the continuous optical monitoring of a fluorogenic PCR and represent a specific application of the well known and commercially available real-time PCR techniques such as Taqman^{®}, Molecular Beacons^{®}, Lightcycler^{®}, Amplifluour^{®} and Scorpion^{®} etc as described in more detail above.

Other nucleic acid amplification techniques may also be modified to detect the methylation status of at least one of the panel of genes, in particular FABP5, which represent novel melanoma markers. Such amplification techniques are well known in the art, and include methods such as NASBA (Compton, 1991 (45)), 3SR (Fahy et al., 1991 (46)) and Transcription Mediated Amplification (TMA). Other suitable amplification methods include the ligase chain reaction (LCR) (Barringer et al, 1990), selective amplification of target polynucleotide sequences (US Patent No. 6,410,276), consensus sequence primed polymerase chain reaction (US Patent No 4,437,975), arbitrarily primed polymerase chain reaction (WO90/06995), nick displacement amplification (W02004/067726). This list is not intended to be exhaustive; any nucleic acid amplification technique may be used provided the appropriate nucleic acid product is specifically amplified.

Sequence variation that reflects the methylation status at CpG dinucleotides in the original genomic DNA offers two approaches to PCR primer design. First, primers that themselves do not cover any potential sites of DNA methylation. Sequence variation at sites of differential methylation are located between the two primers. Such primers are used in bisulphite genomic sequencing, COBRA, Ms-SNuPE. Second, primers that are designed to anneal specifically with either the methylated or unmethylated version of the converted sequence. If there is a sufficient region of complementarity, e.g., 12, 15, 18, or 20 nucleotides, to the target, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues.

One way to distinguish between modified and unmodified DNA is to hybridize oligonucleotide primers which specifically bind to one form or the other of the DNA. After hybridization, an amplification reaction can be performed and amplification products assayed. The presence of an amplification product indicates that a sample hybridized to the primer. The specificity of the primer indicates whether the DNA had been modified or not, which in turn indicates whether the DNA had been methylated or not.
Another way to distinguish between modified and nonmodified DNA is to use oligonucleotide probes which may also be specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

Amplification is achieved with the use of primers specific for the sequence of the gene whose methylation status is to be assessed. In order to provide specificity for the nucleic acid molecules, primer binding sites corresponding to a suitable region of the sequence may be selected. The skilled reader will appreciate that the nucleic acid molecules may also include sequences other than primer binding sites which are required for detection of the methylation status of the gene, for example RNA Polymerase binding sites or promoter sequences may be required for isothermal amplification technologies, such as NASBA, 3SR and TMA.

TMA (Gen-probe Inc.) is an RNA transcription amplification system using two enzymes to drive the reaction, namely RNA polymerase and reverse transcriptase. The TMA reaction is isothermal and can amplify either DNA or RNA to produce RNA amplified end products. TMA may be combined with Gen-probe's Hybridization Protection Assay (HPA) detection technique to allow detection of products in a single tube. Such single tube detection is a preferred method for carrying out the invention.

Thus, in a further embodiment the method of this aspect of the invention is carried out using a technique selected from NASBA, 3SR and TMA.

The methods described above can be used in conjunction with one another to confirm a diagnosis of melanoma including an assessment of how aggressive the melanoma is, and also in prognostic applications, since they provide complementary information about the subject.

It has been shown that treatment with a specific DNA methyltransferase inhibitor leads to re-expression of specific markers associated with melanoma (see International application publication number WO 2006/092610 to University College Dublin, filed 2 March 2006, which reference is incorporated herein in its entirety). These markers include both genes which appear to be regulated directly by methylation and those which are indirectly regulated (see the experimental section for further details). The prior art teaches that Histone deacetylase inhibitors (HDAC) have a similar effect on expression of repressed genes (32, 52 and 53) where methylation causes repression and as a result it is hypothesised that treatment with a HDAC inhibitor may also cause re-expression of specific markers associated with melanoma.

Accordingly, in a third aspect of the invention there is provided a method of treating melanoma in a subject comprising administering a therapeutically effective amount of a DNA methyltransferase inhibitor and/or a histone deacetylase inhibitor to the subject such that expression of FABP5 is increased.

Preferably, the subject is a human. Generally the subject will be one who has been diagnosed with melanoma; for example using the methods according to the present invention.

The method is preferably one carried out to prevent the melanoma from reaching a stage in which it is capable of mestastis. Alternatively, the method may, for example, be used to treat a melanoma which is already in the vertical growth phase (VGP).

In a preferred embodiment, the effect of the treatment is to increase the level of gene expression to the levels of gene expression found in normal melanocytes. However, it may be that any increase in expression will be beneficial for treatment of melanoma. Of course, the DNA methyltransferase inhibitor or histone deacetylase inhibitor is provided in a therapeutically relevant amount to ensure that a controlled increase in gene expression is achieved.

In a further preferred embodiment the therapeutic agent is a DNA methyltransferase inhibitor. The agent may be one which reduces expression of DNMT genes, such as suitable antisense molecules, RNAi molecules or siRNA molecules for example.
In one embodiment, the antisense molecule comprises MG98. Clinical trials by MethylGene have been carried out to study this antisense reagent that specifically inhibits DNMT1 (Reid et al (2002) Selective inhibition of DNA methyltransferase enzymes as a novel strategy for cancer treatment, Curr. Opin. Mol. Ther. 4, 130-137, and see www.methylgene.com for details of phase II clinical trials in metastatic renal cancer patients).

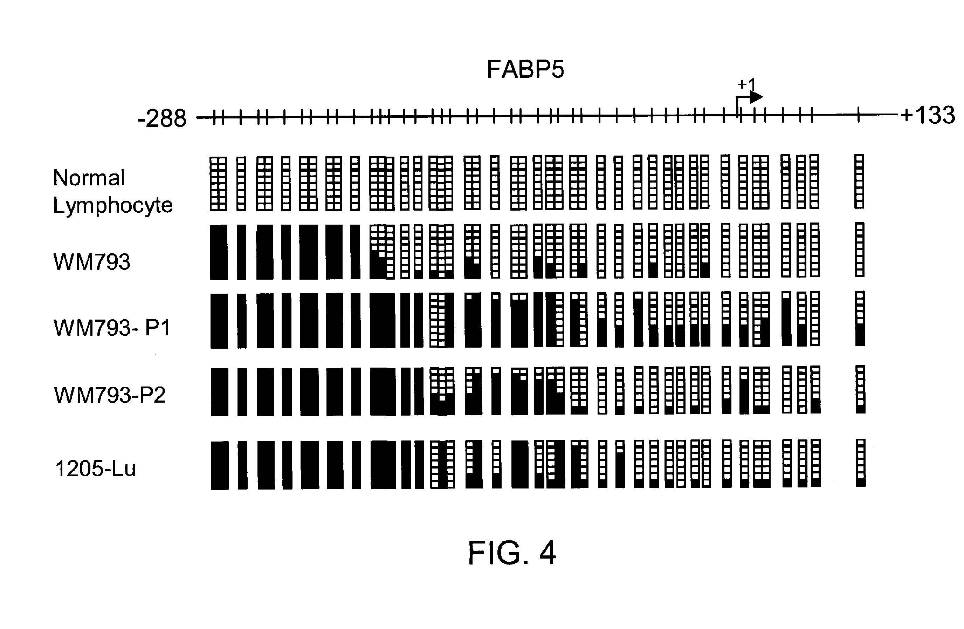

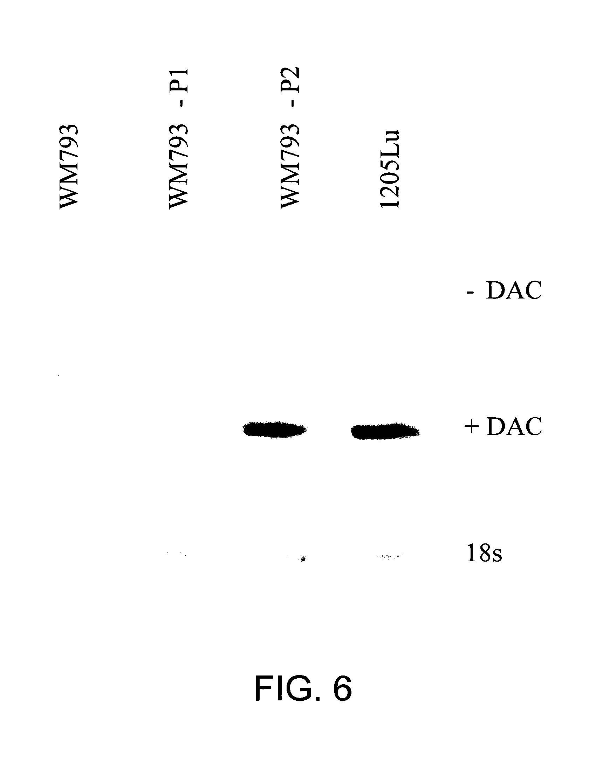

Alternatively, the agent may be a direct inhibitor of DNMTs. Examples include modified nucleotides such as phosphorothioate modified oligonucleotides (fig 6 of ref(68)) and nucleosides and nucleotides such as cytidine analogues. Suitable examples of cytidine analogues include 5-azacytidine, 5-aza-2'-deoxycytidine, 5-fluouro-2'-deoxycytidine, pseudoisocytidine, 5,6-dihydro-5-azacytidine, 1-β-*D*-arabinofuranosyl-5-azacytosine (known as fazabarine) (see figure 4 of ref(68)).

In another embodiment, the DNA methyltransferase inhibitor comprises Decitabine. Full details of this drug can be found at www.supergen.com for example.

Additional DNMT inhibitors include S-Adenosyl-Methionine (SAM) related compounds like ethyl group donors such as L-ethionine and non-alkylating agents such as S-adenosylhomocysteine (SAH), sinefungin, (S)-6-methyl-6-deaminosine fungin, 6-deaminosinefungin, N4-adenosyl-N4-methyl-2,4-diaminobutanoic acid, 5'-methylthio-5'-deoxyadenosine (MTA)and 5'-amino-5'-deoxyadenosine.

Further agents which may alter DNA methylation and which may, therefore, be useful in the present methods and uses include organohalogenated compounds such as chloroform etc, procianamide, intercalating agents such as mitomycin C, 4-aminobiphenyl etc, inorganic salts of arsenic and selenium and antibiotics such as kanamycin, hygromycin and cefotaxim.

However, any suitable DNA methyltransferase inhibitor which is capable of increasing the expression of at least FABP5, and thus can contribute to the treatment of melanoma, is included within the scope of the invention.

In one embodiment, the therapeutic agent is additionally or alternatively a histone deacetylase (HDAC) inhibitor. Preferred non-limiting examples include, trichostatin A (TSA), suberoyl hydroxamic acid (SBHA), 6-(3-chlorophenylureido)caproic hydroxamic acid (3-Cl-UCHA), *m-*carboxycinnamic acid bishydroxylamide (CBHA), suberoylanilide hydroxamic acid (SAHA), azelaic bishydroxamic acid (ABHA), pyroxamide, scriptaid, aromatic sulfonamides bearing a hydroxamic acid group, oxamflatin, trapoxin, cyclic-hydroxamic-acid containing peptides, FR901228, MS-275, MGCD0103 (see www.methylgene.com), short-chain fatty acids and N-acetyldinaline.

Combination therapy using both a DNMT inhibitor and a HDAC inhibitor may give cumulative effects greater than either agent utilised in isolation. Thus, complementary dosage regimes or combination compositions are envisaged in the present invention.

The therapeutic agent may, for example, be encapsulated and/or combined with suitable carriers in solid dosage forms for oral administration which would be well known to those of skill in the art or alternatively with suitable carriers for administration in an aerosol spray. Examples of oral dosage forms include tablets, capsules and liquids.

Alternatively, the therapeutic agent may be administered parenterally. Specific examples include intradermal injection, subcutaneous injection (which may advantageously give slower absorption of the therapeutic agent), intramuscular injection (which can provide more rapid absorption), intravenous delivery (meaning the drug does not need to be absorbed into the blood stream from elsewhere), sublingual delivery (for example by dissolving of a tablet under the tongue or by a sublingual spray), rectal delivery, vaginal delivery, topical delivery, transdermal delivery and inhalation.

In a pharmaceutical composition incorporating a suitable DNA methyltransferase inhibitor or a histone deacetylase, preferred compositions include pharmaceutically acceptable carriers including, for example, non-toxic salts, sterile water or the like. A suitable buffer may also be present allowing the compositions to be lyophilized and stored in sterile conditions prior to reconstitution by the addition of sterile water for subsequent administration. The carrier may also contain other pharmaceutically acceptable excipients for modifying other conditions such as pH, osmolarity, viscosity, sterility, lipophilicity, somobility or the like. Pharmaceutical compositions which permit sustained or delayed release following administration may also be used.

Furthermore, as would be appreciated by the skilled practitioner, the specific dosage regime may be calculated according to the body surface area of the patient or the volume of body space to be occupied, dependent on the particular route of administration to be used. The amount of the composition actually administered will, however, be determined by a medical practitioner based on the circumstances pertaining to the disorder to be treated, such as the severity of the symptoms, the age, weight and response of the individual.

In a fourth aspect of the invention, there is provided the use of a DNA methyltransferase inhibitor or a histone deacetylase inhibitor in the manufacture of a medicament for the treatment of melanoma by increasing expression of FABP5.

The discussion of the third aspect of the invention applies *mutatis mutandis* to this aspect, in particular that the use helps to prevent the melanoma becoming metastatic and/or effectively treats an aggressive (VGP) melanoma.

In a preferred embodiment, the use described above gives rise to a medicament which provides a therapeutic effect wherein the level of gene expression, in particular FABP5 expression, is increased to the levels of gene expression found in normal melanocytes.

Since there are numerous markers for melanoma (for example, as identified in WO 2006/092610) there is benefit in being able to screen for expression of this panel of genes, including FABP5, in a simple and rapid manner. Accordingly, the invention provides, in a fifth aspect, a microarray for use in the methods of the invention which involve determining levels of expression of genes, comprising probes immobilised on a solid support hybridizing with transcripts or parts thereof of FABP5.

Microarrays and their means of manufacture are well known and can be manufactured to order by commercial entities such as Affymetrix, for example.

The probes are the sequences which are immobilized onto the array, by known methods, and which represent selected sequences from the genes of interest, in this case at least the novel marker for melanoma, FABP5. Probe selection and array design lie at the heart of the reliability, sensitivity, specificity, and versatility of the microarrays of the invention. The methods for selecting suitable probes would be readily apparent for one of skill in the art and may involve optimization using data collected from multiple databases, bioinformatics tools, and experiment-trained computer models.

The key elements of probe selection and design are common to the production of all arrays, regardless of their intended application and as such would be well known to one of skill in the art. Strategies to optimize probe hybridization, for example, are invariably included in the process of probe selection. Hybridization under particular pH, salt, and temperature conditions can be optimized by taking into account melting temperatures and using empirical rules that correlate with desired hybridization behaviours.

The GeneChip arrays produced by Affymetrix involve a Perfect Match/Mismatch probe strategy. For each probe designed to be perfectly complementary to a target sequence, a partner probe is generated that is identical except for a single base mismatch in its centre. These probe pairs, called the "Perfect Match probe (PM)" and the "Mismatch probe (MM)", allow the quantitation and subtraction of signals caused by non-specific cross-hybridization. The difference in hybridization signals between the partners, as well as their intensity ratios, serve as indicators of specific target abundance. Such an array design may be applicable to, and incorporated into, the arrays of the present invention.

In order to ensure specificity of the probes in terms of accurately representing the genes listed above, the microarray preferably comprises at least 10 probes representing each gene on the array. However, other numbers of probes may be utilised provided that the expression of each gene which is selected to form part of the array can be accurately and specifically measured.

In a further embodiment, the microarray additionally comprises probes representing transcripts of at least the TSPY1 and/or CYBA and/or MT2A (MT1E) genes, since these genes have previously been shown to be useful in the diagnosis of the progression of melanoma (see International application publication number WO 2006/092610 to University College Dublin, filed 2 March 2006, which reference is incorporated herein in its entirety).

Each probe is preferably at least about 20 nucleotides in length such that a probe of sufficient length to ensure sensitivity and specificity of hybridization is provided. However, any length of probe may be utilised within the scope of the invention, provided that accurate results are achieved in terms of detecting expression of the genes, including FABP5, which represent novel melanoma markers. Possible lengths for the probes include at least 10 nucleotides and up to 250 nucleotides and preferably between about 20 and about 50 nucleotides.

In a similar fashion, the invention also provides a microarray for use in the methods which involve determining the methylation status of the genes representing novel methylation sensitive melanoma markers comprising probes immobilised on a solid support hybridizing with methylated only or both unmethylated and methylated versions of at least the FABP5 gene or a part thereof following bisulphite treatment. Preferably, the probes additionally allow the methylation status of at least TSPY1 to be deteremined. In an additional embodiment, the probes further allow the methylation status of at least CYBA and/or MT2A (MT1E) to be determined.

As discussed above, bisulphite treatment of nucleic acid will convert unmethylated cytosine residues to uracil, but methylated cytosine residues (found at specific CpG sites) will not be converted. Thus, it is possible to design probes which will discriminate between methylated (remain as cytosine following bisulphite treatment) and unmethylated (converted to uracil) sites in terms of target hybridisation.

In one embodiment, the probes correspond to appropriate portions of the promoter the FABP5 gene, as this region has been shown to be increasingly methylated in more invasive melanoma cell lines.

Thus, the microarray may contain two sets of probes, one which is specific for genes methylated at particular sites and one specific for genes which are unmethylated at those particular sites. Preferably, each probe will represent only one potential site of methylation to allow full specificity of binding and detection for each methylation site, although this is not essential.

Alternatively, the microarray may be a "methylation specific" array in that it contains probes designed to solely bind targets which are methylated at the specific CpG sites in the relevant genes and thus are protected from the effects of bisulphite treatment. Such an array could be utilised in conjunction with an array which comprises probes specific only for targets which are unmethylated at specific CpG sites. The same sample may be utilised with each array in order to determine relative levels of methylation.

Following bisulphite treatment of the sample, optionally followed by amplification (see above for details of the MSP technique etc.), the target nucleic acid is hybridised to the array in order to determine whether the genes of interest are methylated at specific sites. The positive identification of methylation is indicative of melanoma or a more invasive/aggressive form of melanoma (such as VGP melanoma).

Either the probes or the target, or both, may be labelled with a suitable label to allow detection of hybridization of the target to its complementary probe. The most preferred type of label is a fluorescent label although other labels such as radiolabels and mass-labels may also be utilised.

The tissue sample to be used is as described above and the subject from which the sample containing the test (target) nucleic acid is derived is most preferably a human subject.

By identifying the novel marker, FABP5, linked to melanoma, new treatments for this disease may be discovered. Thus, the expression pattern linked to melanoma and in particular progression of melanoma to more invasive/aggressive (metastatic) forms may be used as a research tool to identify new pharmaceuticals which may be used to treat, prevent or control melanoma.

Accordingly, in a further aspect the invention provides an *in vitro* method of identifying a compound capable of treating or reducing the effects or progression of melanoma comprising the steps of;
(a) administering the compound to a melanoma sample;
(b) generating an expression profile of FABP5 ;
(c) comparing the expression profile obtained in (b) with the expression profile of the corresponding gene, expressed in a control melanocyte sample which is not a melanoma;
wherein a positive correlation of the expression profiles is indicative that the compound is capable of reducing, controlling the progression of, or preventing melanoma.

Similarly, the invention also provides a similar compound screening and lead identification method which involves use of non-human animals; in particular a method of identifying a compound capable of treating or reducing the effects or progression of melanoma comprising the steps of;
(a) administering the compound to an experimental non-human animal having a melanoma;
(b) generating an expression profile of FABP5;
(c) comparing the expression profile obtained in (b) with the expression profile of the corresponding gene, expressed in a control melanocyte sample which is not a melanoma;
wherein a positive correlation of the expression profiles is indicative that the compound is capable of reducing the effects of, preventing or preventing progression of melanoma.

The experimental animal may be sacrificed following the testing.

In particular, these methods allow leads to be identified which either are effective in preventing melanoma from reaching a metastatis form and/or which are capable of regressing a melanoma from an aggressive phase, such as VGP, to a less dangerous form of melanoma.

In one embodiment, the control sample is taken from an experimental non-human animal which does not have a melanoma. Preferably, the control sample is taken from normal melanocytes in the same non-human animal.

FABP5 is the most preferred gene for inclusion in the panels. Preferred gene panels include at least TSPY1 and/or CYBA and/or MT2A (MT1E) in addition to FABP5.

The invention also provides the compounds identified by the methods described above. These compounds may be formulated into suitable pharmaceutical compositions, including suitable carriers, for administration to a patient in need thereof. Details of such compositions are provided above, and it is thought that some of the specific DNMT and HDAC inhibitors may prove to act positively in the screening methods according to this aspect of the invention.

In addition, there is also provided a method of treating melanoma comprising administering to a subject in need thereof a therapeutically effective amount of a compound which has been identified using the screening methods of the invention.

The invention will now be further described, by way of example, with reference to the following experimental section and figures in which:

### BRIEF DESCRIPTION OF THE FIGURES

Figure 1 shows the nucleotide sequence of the FABP5 gene promoter. The first and second exons are presented in capitals. The CpG island is shown in a lighter shade than the remainder of the nucleotide sequence.
Figure 2 shows the bisulphite sequencing primers utilised for determining the methylation status in the promoter region of FABP5.
Figure 3 presents results of bisulphite sequencing analysis. The figure includes a schematic representation of CpG dinucleotides in the promoter region and first exon of analysed FABP5 gene in the WM793 series. White ovals indicate unmethylated residues and black ovals represent methylated residues. Arrow indicates approximate start site.
Figure 4 presents further results of bisulphite sequencing analysis. The figure includes a schematic representation of CpG dinucleotides in the promoter region and first exon of analysed FABP5 gene in the WM793 series and in 1205-Lu cell lines as compared to normal lymphocytes. Black squares represent methylation and white squares correspond to a status of no methylation.
Figure 5 presents results of methylation specific PCR carried out for the FABP5 gene on melanoma biopsies.
Figure 6 shows the effect of DAC treatment on FABP5 expression in melanoma cell lines. Total RNA was extracted from untreated (- DAC) and treated (+ DAC) cells and subjected to RT-PCR analysis via a cycle limitation method. Order of parental WM793 and derivatives are indicated in the figure.

### EXPERIMENTAL SECTION

### Identification of FABP5 as a marker for melanoma progression

Reference is made to Gallagher et al., Carcinogenesis 2005, 26(11), 1856-1867 for full details of the original analysis of gene expression to determine markers for melanoma. This document also provides full details of the relevant cell lines and techniques utilised. The reported analysis was repeated using Affymetrix Microarray Suite (MAS) version 5.0. The new results reveal a correlation between expression of FABP5 and the invasiveness of the cell lines.

### FABP5 gene analysis

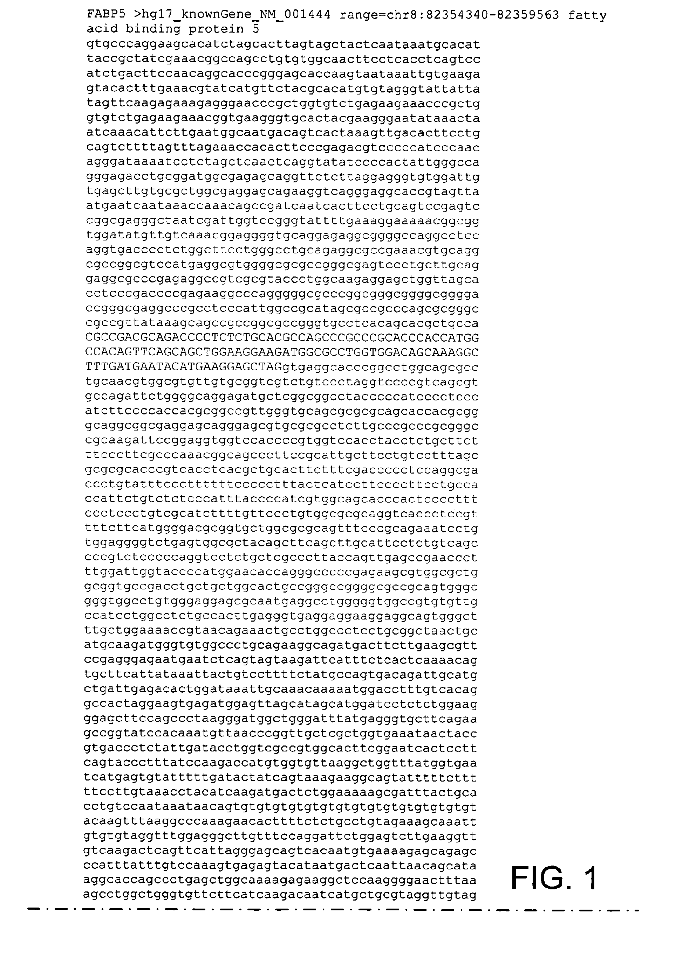

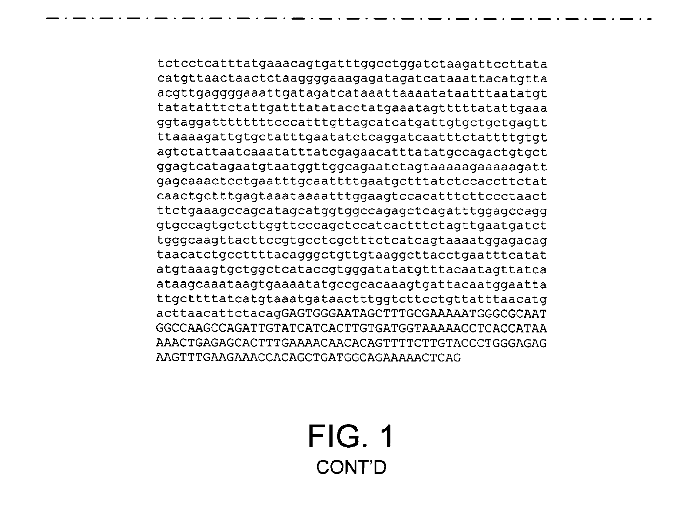

The nucleotide sequence of the promoter region of the FABP5 gene, including the first and second exons is set forth in figure 1 (SEQ ID NO: 1). The CpGPLOT software program (http://bioinfo.hku.hk/cgi-bin) and the CpG Island Searcher software (see http://cpgislands.usc.edu/ and Takai D, Jones PA. The CpG island searcher: a new WWW resource. In Silico Biol. 3, 235-240 (2003)) were use to locate the CpG island and the CpG island is highlighted in the figure in lighter shading than the flanking nucleotide sequences.

### Bisulphite sequencing of gene promoter regions.

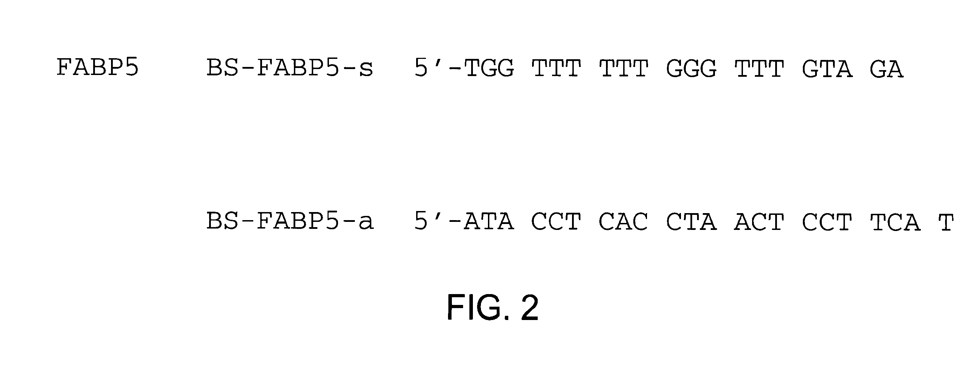

Bisulphite sequencing was carried out for the FABPB gene to determine whether there was differential methylation in the more invasive derivative cells compared to the parental cells. The primers utilised are presented in figure 2 and the protocol utilised was as follows:

### Bisulphite Modification

Put one heating block at 37°C and another at 50°C. Make reagents fresh each time.

### REAGENTS

- Sodium bisulphite 4.3M pH 5 [S9000/500g/Minimum 99% SIGMA]. Mr125.33

o Dissolve 7.6g in 20ml dH₂O. Add 500µl 10M NaOH. This should give a solution of pH 5.

- Hydroquinone 20mM [H9003/100g/99+% SIGMA]

o Hydroquinone is photosensitive so wrap tube in tinfoil prior to beginning. Dissolve 90mg in 50ml dH₂O.

- NaOH 3M

o Dissolve 1.2g in 10ml dH₂O.

### DNA MODIFICATION WITH SODIUM BISULPHITE

- Dilute 1µg DNA in 50µl H₂O in an eppendorf.
- Add 5.7µl 3M NaOH to each sample and incubate for 10-15 mins at 37°C.

o Resulting solution contains ssDNA sensitive to bisulphite.

- Add 33µl 20mM hydroquinone. This is an antioxidant that prevents intermediate oxidation. Solution should turn yellow.
- Add 530µl 4.3M sodium bisulphite pH 5 to each sample. Mix well and incubate at 50°C for 16-17 hrs. Ensure the tubes are in darkness. Between 16 and 17 hours is optimal for this reaction and as such the incubation time should be strictly adhered to.

### PURIFICATION AND PRECIPITATION OF MODIFIED DNA

### Promega Wizard DNA Clean-Up System Using Vacuum Manifold (catalogue no: A7280).

- Title both syringes and columns, and attach to manifold.
- Prepare vacuum system by blocking unused lanes, closing fluxes and vacuum.
- Insert syringes.
- Add 1 ml resin to eppendorfs containing modified DNA.
- Mix resin before use. (Guanidine thiosianate)
- Add mix to syringes.
- Open vacuum and then flux. Once the resin has passed, close fluxes and vacuum to prevent drying out.
- Add 3ml 80% isopropanol and allow to flow through.
- Repeat with 1ml 80% isopropanol.
- Discard syringes and add columns to old eppendorfs. Centrifuge at 13,000rpm for 1 min to remove trace isopropanol.
- Add columns to a new eppendorf and add 50µl H₂O. Allow to stand for 3 mins at room temp.
- Centrifuge at 13,000rpm for 1 min. DNA is in eluate.
- Add 5.7µl 3M NaOH to tubes and incubate for 15-20 mins at 37°C. This is to complete the chemical conversion of C to U and eliminate salts.
- Thaw glycogen (20mg/ml).
- Make a master mix of 0.5µl glycogen (20mg/ml) with 17µl 10M ammonium acetate, per sample.
- Add glycogen mix to each tube and 3 vols of ice cold absolute ethanol. Incubate at -70°C overnight in darkness.
- Following incubation, centrifuge at 12,000rpm for 20 mins at 4°C.
- Discard the supernatant, wash with 1ml of ice cold 70% ethanol, and centrifuge at 4°C for 20 mins.
- Discard supernatant and dry pellet (speedvac: 10 mins at 45°C).
- Resuspend with 25µl H₂O.

Final solution should be at a concentration of 25ng/µl. 50ng (2µl) is used in the PCR.

Following PCR, the band is excised and purified using the QIAquick Gel Extraction Kit (Qiagen).

Cloning of PCR Products in Smaller Volume (using the Promega pGEM-T Easy vector system II; Cat. # A1380)

### LIGATION

- Keep everything on ice.
- Briefly centrifuge the pGEM vector and Control Insert DNA tubes
- Add 1.5µl PCR product to each tube.
- Prepare mix (per tube)
- 2X ligation buffer 2.5
- pGEM T vector 0.5
- T4 DNA ligase 0.5
- Add 3.5µl of mix to each tube.
- Incubate at 15°C for 3 hours or at 4°C overnight. (4°C O/N gives max number of transformants.)

### TRANSFORMATION

- Keep everything on ice.
- Centrifuge ligation products briefly.
- Take the competent cells from -80°C and put on ice until thawed.
- Transfer 25µl of bacterial cells to ligation products tube.
- Incubate on ice for 30 mins.
- Heat-shock the samples at 42°C for 1 min.
- Immediately return the tubes to ice for 2 mins.
- Add 600µl room temp LB medium.
- Incubate at 37°C for 1 hr with shaking (400 rpm).
- Meanwhile, take LB/Amp plates from cold room. Incubate plates for about 10 mins at 37°C to ensure they are not cold.
- Prepare the following mixture (per plate):
   o DiMethylFormamide (AppliedChem-A3676-0500) 80µl
   o X-Gal 50mg/ml 20µl
   o IPTG 1M 20µl
- Apply 120µl of mix to each plate.
- Following incubation, centrifuge the samples for 1 min at 4000 rpm.
- Discard 500µl of supernatant (to concentrate the bacteria) and plate samples with 60 µl of the transformation product.
- Incubate at 37°C overnight.
   MINI-PREP
- Put plates in the fridge for 1 hr to stop growth and finalise colour.
- Add 4ml LB/Amp (1µl/ml) to a 15ml falcon.
- Pick the colonies with a sterile pipette tip and add to the falcons.
- Incubate overnight at 37°C.
- Mini prep is then carried out using the QIAprep Spin Miniprep Kit (Qiagen) and the product sequenced.

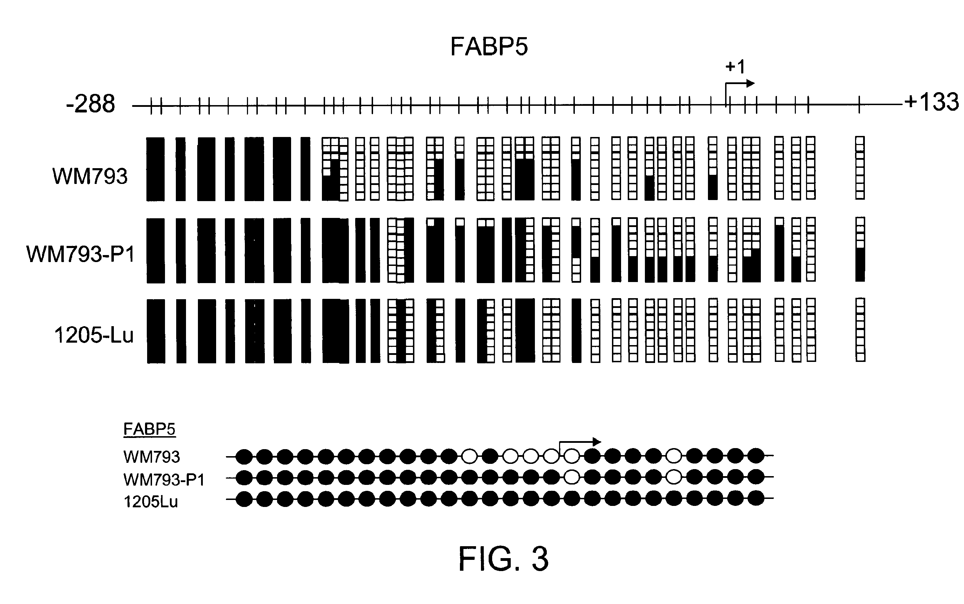

As can be seen from the results presented in figure 3, FABP5 is methylated to a degree in the parental cells (WM793) and becomes increasingly methylated in the more aggressive derivatives (WM793-P1 and 1205-Lu). Increased methylation of the FABP5 gene thus appears to have an important impact on melanoma progression.

Further results including all of the WM793 cell line derivatives (which become increasingly aggressive in order WM793 < WM793-P1 < WM793-P2) together with results for a separate melanoma cell line 1205-Lu are presented in Figure 4. Also shown in this figure is the methylation status of lymphocytes as a normal tissue control. The results presented in this figure are also consistent with the importance of increasing levels of methylation in the FABP5 promoter in predicting the aggressiveness of melanoma.

### Methylation Specific PCR (MSP)

Methylation-specific PCR (MSP) was performed on a range of 28 matched primary and metastatic melanomas for FABP5. MSP for FABP5 had 37 informative samples of which 9 were methylated and 28 were not methylated at the specific site analysed.

Results of methylation specific PCR carried out for the FABP5 gene on melanoma biopsies are shown in figure 5. Completion of this sample cohort, along with additional samples and statistical analysis may reveal the importance of methylation of this gene in melanoma.

MSP was carried out according to standard protocols, as are well known in the art. Reference can be made to Herman et al (65) and see WO 97/46705, both of which are incorporated herein by reference, for example.

The MSP Primers utilised were as follows:

### FABP5

Methylated MSP-FABP5-sm TTT GGG TTT GTA GAG GCG TC (SEQ ID NO: 4)
MSP-FABP5-am TTA CCA AAA TAC GCG ACG AC (SEQ ID NO: 5)
Unmethylated MSP-FABP5-su TTT TTT GGG TTT GTA GAG GTG TT (SEQ ID NO: 6)
MSP-FABP5-au ACC AAA ATA CAC AAC AAC CTC TC (SEQ ID NO: 7)

### RT-PCR Analysis via Cycle Limitation to assess affect of DAC treatment

RNA extracts were pre-digested with DNase I prior to cDNA synthesis using the DNA-Free kit (Ambion). Single-stranded cDNA was synthesized from 1 µg total RNA using the ImProm-II Reverse Transcription kit (Promega). Recombinant RNasin Ribonuclease Inhibitor (20 U/20 µL reaction; Promega) was added to prevent RNase-mediated degradation. Two negative controls were also utilised, namely minus reverse transcriptase enzyme control and minus template control. Following inactivation of reverse transcriptase (RT) at 70°C for 15 minutes, aliquots of generated single-stranded cDNA were subjected to polymerase chain reaction (PCR) amplification via a cycle limitation approach.

18s was used as a control gene whose expression is unaffected by DAC treatment.

### Primers used were as follows:

FABP5-For: TCA GCA GCT GGA AGG AAG AT (SEQ ID NO: 8)
FABP5-Rev: GCC ATC AGC TGT GGT TTC TT (SEQ ID NO: 9)

As shown in figure 6, treatment of the derivative cell lines with DAC lead to increased expression of the FABP5 gene. The results indicate that methylation of the FABP5 gene increases in the more aggressive melanoma cell lines, since DAC treatment recovered greater expression in these cells than in the parental melanoma cell line (WM793).

### REFERENCES

1. Tucker, M.A. and Goldstein, A.M. (2003) Melanoma etiology: where are we? Oncogene, 22, 3042-52.
2. Beddingfield, F.C (2003) The melanoma epidemic: res ipsa loquitur. Oncologist, 8,459-65.
3. Chin, L., Merlino, G. and DePinho, R.A. (1998) Malignant melanoma: modern black plague and genetic black box. Genes Dev, 12, 3467-81.
4. Chung, C.H., Bernard, P.S. and Perou, C.M. (2002) Molecular portraits and the family tree of cancer. Nat Genet, 32, Suppl533-40.
5. Baldi, A., Santini, D., Uca Ade, L., and Paggi, M.G. (2003) cDNA array technology in melanoma: an overview. J Cell Physiol, 196, 219-23.
6. Bittner, M., Meltzer, P., Chen, Y. et al. (2000) Molecular classification of cutaneous malignant melanoma by gene expression profiling. Nature, 406, 536-40.
7. Clark, E.A., Golub, T.R., Lander, E.S. and Hynes, R.O. (2000) Genomic analysis of metastasis reveals an essential role for RhoC. Nature, 406, 532-5.
8. Su, Y.A., Bittner, M.L., Chen, Y. et al. (2000) Identification of tumor-suppressor genes using human melanoma cell lines UACC903, UACC903(+6), and SRS3 by comparison of expression profiles. Mol Carcinog, 28, 119-27.
9. Certa, U., Seiler, M., Padovan, E. and Spagnoli, G.C. (2001) High density oligonucleotide array analysis of interferon-alpha2a sensitivity and transcriptional response in melanoma cells. Br J Cancer, 85, 107-14.
10. Seftor, E.A., Meltzer, P.S., Kirschmann, D.A., et al. (2002) Molecular determinants of human uveal melanoma invasion and metastasis. Clin Exp Metastasis, 19, 233-46.
11. Wang, E., Miller, L.D., Ohnmacht, G.A. et al. (2002) Prospective molecular profiling of melanoma metastases suggests classifiers of immune responsiveness. Cancer Res, 62, 3581-6.
12. Baldi, A., Battista, T., De Luca, A. et al. (2003) Identification of genes down-regulated during melanoma progression: a cDNA array study. Exp Dermatol, 12, 213-8.
13. van der Velden, P.A., Zuidervaart, W., Hurks, M.H. et al. (2003) Expression profiling reveals that methylation of TIMP3 is involved in uveal melanoma development. Int J Cancer, 106, 472-9.
14. Hsu, M-Y., Elder, D.E. and Herlyn, M. (1999) Melanoma: the Wistar (WM) melanoma cell lines. In: JRW Masters and B Palsson (eds.), Human Cell Culture, pp. 259-274. London: Kluwer Acad Publ,.
15. Kobayashi, H., Man, S., MacDougall, J.R., Graham, C.H., Lu, C. and Kerbel, R.S. (1994) Variant sublines of early-stage human melanomas selected for tumorigenicity in nude mice express a multicytokine-resistant phenotype. Am J Pathol, 144, 776-86.
16. Juhasz, I., Albelda, S.M., Elder, D.E. et al. (1993) Growth and invasion of human melanomas in human skin grafted to immunodeficient mice. Am J Pathol, 143, 528-37.
17. Easty, D.J., Mitchell, P.J., Patel, K., Florenes, V.A., Spritz, R.A. and Bennett DC. (1997) Loss of expression of receptor tyrosine kinase family genes PTK7 and SEK in metastatic melanoma. Int J Cancer, 71, 1061-5.
18. McArdle, L., Rafferty, M., Maelandsmo, G.M. et al. (2001) Protein tyrosine phosphatase genes downregulated in melanoma. J Invest Dermatol, 117, 1255-60.
19. Breen, C.J., Barton, L., Carey, A. et al. (1999) Applications of comparative genomic hybridization in constitutional chromosome studies. J Med Genet, 36, 511-7.
20. Dasari, V.K., Deng, D., Perinchery, G., Yeh, C.C. and Dahiya, R.D. (2002) DNA methylation regulates the expression of Y chromosome specific genes in prostate cancer. J Urol, 167, 335-8.
21. Fragra, M.F., Uriol, E., Borja Diego, L., et al. (2002) High-performance capillary electrophoretic method for the quantification of 5-methyl 2'-deoxycytidine in genomic DNA: application to plant, animal and human cancer tissues. Electrophoresis, 23, 1677-81.
22. Jin, H., Yang, R., Awad, T.A. et al. (2001) Effects of early angiotensin-converting enzyme inhibition on cardiac gene expression after acute myocardial infarction. Circulation, 103, 736-42.
23. Southern, E.M. (1975) Detection of specific sequences among DNA fragments separated by gel electrophoresis. J Mol Biol, 98, 503-17.
24. Kath, R., Jambrosic, J.A., Holland, L., Rodeck, U. and Herlyn M. (1991) Development of invasive and growth factor-independent cell variants from primary human melanomas. Cancer Res, 51, 2205-11.
25. Balaban, G., Herlyn, M., Guerry, D. 4th, et al. (1984) Cytogenetics of human malignant melanoma and premalignant lesions. Cancer Genet Cytogenet, 11, 429-39.
26. Balaban, G.B., Herlyn, M., Clark, W.H. Jr and Nowell, P.C. (1986) Karyotypic evolution in human malignant melanoma. Cancer Genet. Cytogenet, 19, 113-22.
27. Hildenbrand, R., Schroder, W., Brude, E., Schepler, A., Konig, R., Stutte, H.J. and Arnemann, H. (1999) Detection of TSPY protein in a unilateral microscopic gonadoblastoma of a Turner mosaic patient with a Y-derived marker chromosome. J Pathol, 189, 623-6.
28. Lau, Y.F. (1999) Gonadoblastoma, testicular and prostate cancers, and the TSPY gene. Am J Hum Genet, 64, 921-7.
29. Lau, Y. and Zhang, J. (2000) Expression analysis of thirty one Y chromosome genes in human prostate cancer. Mol Carcinog, 27, 308-21.
30. Lau, Y.F., Lau, H.W. and Komuves, L.G. (2003) Expression pattern of a gonadoblastoma candidate gene suggests a role of the Y chromosome in prostate cancer. Cytogenet Genome Res, 101, 250-260.
31. Liang, G., Gonzales, F.A., Jones, P.A., Orntoft, T.F. and Thykjaer, T. (2002) Analysis of gene induction in human fibroblasts and bladder cancer cells exposed to the methylation inhibitor 5-aza-2'-deoxycytidine. Cancer Res, 61, 961-6.
32. Plumb, J.A., Strathdee, G., Sludden, J., Kaye, S.B. and Brown, R. (2000) Reversal of drug resistance in human tumor xenografts by 2'-deoxy-5-azacytidine-induced demethylation of the hMLH1 promoter. Cancer Res, 60, 6039-44.
33. Krick, R., Jakubiczka, S. and Arnemann, J. (2003) Expression, alternative splicing and haplotype analysis of transcribed testis specific protein (TSPY) genes. Gene, 302, 11-9.
34. Arnemann, J., Jakubiczka, J., Thuring, S. and Schmidtke, J. (1991) Cloning and seqeunce analysis of a human Y-chromosome-derived, testicular cDNA, TSPY. Genomics, 11, 108-14.
35. Schneiders, F., Dork, T., Arnemann, J., Vogel, T., Werner, M. and Schmidtke, J. (1996) Testis-specific protein, Y-encoded (TSPY) expression in testicular tissues. Hum Mol Genet, 5, 1801-7.
36. Zhang, J.S., Yang-Feng, T.L., Muller, U., Mohandas, T.K., de Jong, P.J. and Lau, Y.F. (1992) Molecular isolation and characterization of an expressed gene from the human Y chromosome. Hum Mol Genet, 1, 717-26.
37. Dasari, V.K., Goharderakhshan, R.Z., Perinchery, G., et al. (2001) Expression analysis of Y chromosome genes in human prostate cancer. J Urol, 165, 1335-41.
38. Baylin, S.B., Herman, J.G., Graff, J.R., Vertino, P.M. and Issa, J.P. (1998) Alterations in DNA methylation: a fundamental aspect of neoplasia. Adv Cancer Res, 72, 141-96.
39. Herman, J.G. and Baylin, S.B. (2003) Gene silencing in cancer in association with promoter hypermethylation. N Engl J Med, 349, 2042-54.
40. Momparler, R.L. (2003) Cancer epigenetics. Oncogene, 22, 6479-83.
41. van der Velden, P.A., Metzelaar-Blok, J.A., Bergman, W., et al. (2001) Promoter hypermethylation: a common cause of reduced p16(INK4a) expression in uveal melanoma. Cancer Res, 61, 5303-06.
42. Zhou, X.P., Gimm, O., Hampel, H., Niemann, T., Walker, M.J. and Eng, C. (2000) Epigenetic PTEN silencing in malignant melanomas without PTEN mutation. Am J Pathol, 157, 1123-8.
43. Soengas, M.S., Capodieci, P., Polsky, D., et al. (2001) Inactivation of the apoptosis effector Apaf-1 in malignant melanoma. Nature, 409, 207-11.
44. Karpf, A.R., Lasek, A.W., Ririe, T.O., Hanks, A.N., Grossman, D. and Jones, D.A. (2004) Limited gene activation in tumor and normal epithelial cells treated with the DNA methyltransferase inhibitor 5-aza-2'-deoxycytidine. Mol Pharmacol, 65, 18-27.
45. Zendman, A.J., van Kraats, A.A., den Hollander, A.I., Weidle, U.H., Ruiter, D.J. and van Muijen, G.N. (2002) Characterization of XAGE-1b, a short major transcript of cancer/testis-associated gene XAGE-1, induced in melanoma metastasis. Int J Cancer, 97, 195-204.
46. Serrano, A., Tanzarella, S., Lionello, I., et al. (2001) Re-expression of HLA class I antigens and restoration of antigen-specific CTL response in melanoma cells following 5-aza-2'-deoxycytidine treatment. Int J Cancer, 94, 243-51.
47. Fulda, S., Kufer, M.U., Meyer, E., van Valen, F., Dockhorn-Dworniczak, B. and Debatin, K.M. (2001) Sensitization for death receptor- or drug-induced apoptosis by re-expression of caspase-8 through demethylation or gene transfer. Oncogene, 20, 5865-77.
48. Suzuki, H., Gabrielson, E. and Chen, W. (2002) A genomic screen for genes upregulated by demethylation and histone deacetylase inhibition in human colorectal cancer. Nat Genet, 31, 141-9.
49. Shi, H., Wei, S.H., Leu, Y., et al. (2003) Triple analysis of the cancer epigenome: an integrated microarray system for assessing gene expression, DNA methylation, and histone acetylation. Cancer Res, 63, 2164-71.
50. Jones, P.A. and Baylin, S.B. (2002) The fundamental role of epigenetic events in cancer. Nat Rev Genet, 3, 415-28.
51. Brown, R. and Strathdee, G. (2002) Epigenomics and epigenetic therapy of cancer. Trends Mol Med, 8, S43-8.
52. Smith, K.S., Folz, B.A., Adams, E.G. and Bhuyan, B.K. (1995) Synergistic and additive combinations of several antitumor drugs and other agents with potent alkylating agent adozelsin. Cancer Chemother Pharmacol, 35, 471-82.
53. Kozar, K., Kaminski, R., Switaj, T., et al. (2003) Interleukin 12-based immunotherapy improves the antitumour effectiveness of a low-dose 5'-aza-2'-deoxycitidine treatment in L1210 leukemia and B16F10 melanoma models in mice. Clin Cancer Res, 9, 3124-33.
54. Abele, R., Clavel, M., Dodion, P., et al. (1987) The EORTC Early Clinical Trials Cooperative Group experience with 5-aza-2'-deoxycytidine (NSC 127716) in patients with colo-rectal, head and neck, renal carcinomas and malignant melanomas. Eur J Cancer Clin Oncol, 23, 1921-4.
55. Anzai, H., Frost, P. and Abbruzzese, JL. (1992) Synergistic cytotoxicity with 2'-deoxy-5-azacytidine and topotecan in vitro and in vivo. Cancer Res, 52, 2180-5.
56. Coral, S., Sigalotti, L., Gasparollo, A., Cattarossi, I., Visintin, A. and Cattelan, A. (1999) Prolonged upregulation of the expression of HLA class I antigens and costimulatory molecules on melanoma cells treated with 5-aza-2'-deoxycytidine (5-AZA-CdR). J Immunother, 22, 16-24.
57. Wang, E., Miller, L.D., Ohnmacht, G.A., et al. (2002) Prospective molecular profiling of melanoma metastases suggests classifiers of immune responsiveness. Cancer Res, 62, 3581-6.
58. Gibson U. E. M, Heid C. A. and Williams, P. M. A novel method for real time quantitative RT-PCR. Genome Research 6:995-1001 (1996).
59. Holland et al; Detection of specific polymerase chain reaction product by utilising the 5'-3' exonuclease activity of Thermus aquaticus DNA polymerase; Proc. Natl. Acad. Sci. USA 88, 7276-7280 (1991).
60. Gelmini et al. Quantitative polymerase chain reaction-based homogeneous assay with flurogenic probes to measure C-Erbb-2 oncogene amplification. Clin. Chem. 43, 752-758 (1997).
61. Livak et al. Towards fully automated genome wide polymorphism screening. Nat. Genet. 9, 341-342 (1995).
62. Tyagi & Kramer. Molecular beacons - probes that fluoresce upon hybridization. Nat. Biotechnol. 14, 303-308 (1996).
63. Tyagi et al. Multicolor molecular beacons for allele discrimination. Nat. Biotechnol. 16, 49-53 (1998).
64. David Whitcombe, Jane Theaker, Simon P. Guy, Tom Brown, Steve Little. Detection of PCR products using self-probing amplicons and fluorescence; Nature Biotechnology 17, 804 - 807 (01 Aug 1999) 11.
65. Herman JG, Graff JR, Myohanen S, Nelkin BD, Baylin SB. Methylation-specific PCR: a novel PCR assay for methylation status of CpG islands. Proc Natl Acad Sci U S A 1996; 93(18):9821-9826.
66. Rein T, DePamphilis M L, Zorba H. Nucleic acids research, 1998, Vol. 26, No. 10, 2255-2264.
67. Nazarenko I. A., Bhatnagar S. K. And Hohman R.J. Nucleic Acids Research, 1997, Vol. 25, No. 12, 2516-2521.
68. Villar-Garea, A and Esteller, M. Current Drug Metabolism, 2003, 4, 11-31.

## Claims

1. A method of diagnosing melanoma and/or for monitoring melanoma progression, in particular for determining whether the melanoma is likely to have metastatic capabilities, in a subject comprising, in a test sample, determining the methylation status of FABP5 wherein methylation of the gene indicates a positive diagnosis of melanoma and/or increased methylation of the gene indicates the progression of melanoma.

2. A method according to claim 1 wherein the test sample is a sample taken from tissue which is suspected of being a melanoma.

3. The method according to claim 1 or 2 wherein the methylation status of at least TSPY1 is also determined.

4. The method according to any of claims 1 to 3 wherein the methylation status of at least CYBA and/or MT2A is also determined.

5. The method according to any one of claims 1 to 4 which utilises methylation specific PCR.

6. The method according to claim 5 which utilises real-time quantitative methylation specific PCR.

7. A method of diagnosing melanoma and/or for monitoring melanoma progression, in particular for determining whether the melanoma is likely to have metastatic capabilities, in a subject comprising, in a test sample, determining expression levels of FABP5, wherein a low level of expression indicates a positive diagnosis of melanoma and wherein a statistically significant decrease in the level of expression of FABP5 indicates the progression of melanoma in the subject.

8. The method according to claims 7 wherein the expression of at least TSPY1 is also determined.

9. The method according to claim 7 or 8 wherein the expression of at least CYBA and/or MT2A is also determined.

10. The method according to any one of claims 7 to 9 wherein gene expression is determined using RT-PCR, preferably quantitative RT-PCR.

11. The method according to any one of claims 1 to 10 which is used to determine whether the melanoma is in early radial growth phase or a subsequent vertical growth phase.

12. The method according to any one of claims 1 to 11 which is used to determine the appropriate treatment for the subject.

13. The method according to anyone of claims 1 to 12 wherein the methylation status or level of expression is assessed with reference to a control sample.

14. The method according to claim 13 wherein the control sample is taken from normal melanocytes in the subject.

15. The method according to claim 13 wherein the control sample is taken from the same tissue as the test sample at an earlier time point.

16. A method of treating melanoma in a subject comprising administering a therapeutically effective amount of a DNA methyltransferase inhibitor or a histone deacetylase inhibitor to the subject such that expression of FABP5 is increased.

17. The method according to claim 16 wherein the level of gene expression is increased to the levels of gene expression found in normal melanocytes.

18. Use of a DNA methyltransferase inhibitor or a histone deacetylase inhibitor in the manufacture of a medicament for the treatment of melanoma by increasing expression of FABP5.

19. The use according to claim 18 wherein the level of gene expression is increased to the levels of gene expression found in normal melanocytes.

20. A microarray for use in the method of any one of claims 7 to 10 and 11 to 15 when dependent from any one of claims 7 to 10 comprising probes immobilised on a solid support hybridizing with transcripts or parts thereof of FABP5.

21. The microarray of claim 20 which further comprises probes representing transcripts of TSPY1.

22. The microarray of claim 20 or 21 which further comprises probes representing transcripts of CYBA and/or MT2A.

23. A microarray for use in the method of any one of claims 1 to 6 and 11 to 15 when dependent from any one of claims 1 to 6 comprising probes immobilised on a solid support hybridizing with either methylated only or both unmethylated and methylated versions of FABP5 following bisulphite treatment.

24. The microarray of claim 23 which further comprises probes hybridizing with either methylated only or both unmethylated and methylated versions of TSPY1.

25. The microarray of claim 20 or 21 which further comprises probes hybirdizing with either methylated only or both unmethylated and methylated versions of CYBA and/or MT2A.

26. A method of identifying a compound capable of treating or reducing the effects or progression of melanoma comprising the steps of;
(a) administering the compound to an experimental non-human animal having a melanoma;
(b) generating an expression profile of FABP5 ;
(c) comparing the expression profile obtained in (b) with the expression profile of the corresponding gene, expressed in a control melanocyte sample which is not a melanoma;
wherein a positive correlation of the expression profiles is indicative that the compound is capable of reducing the effects or progression of melanoma or preventing melanoma.

27. An *in vitro* method of identifying a compound capable of treating or reducing the effects or progression of melanoma comprising the steps of;
(a) administering the compound to a melanoma sample;
(b) generating an expression profile of FABP5;
(c) comparing the expression profile obtained in (b) with the expression profile of the corresponding FABP5 gene, expressed in a control melanocyte sample which is not a melanoma;
wherein a positive correlation of the expression profiles is indicative that the compound is capable of reducing, controlling the progression of, or preventing melanoma.

28. The method of claim 26 wherein the control sample is taken from an experimental non-human animal which does not have a melanoma.

29. The method of claim 26 wherein the control sample is taken from normal melanocytes in the same non-human animal.


SEQUENCE LISTING

<110>  Oncomethylome Sciences SA

<120>  Markers for Melanoma Progression

<130>  P83993EP00

<150>  PCT/GB2006/000756
<151>  2006-03-02

<150>  GB 0604370.7
<151>  2006-03-03

<160>  9

<170>  PatentIn version 3.3

<210>  1
<211>  3989
<212>  DNA
<213>  Homo sapiens

<400>  1
gtgcccagga agcacatcta gcacttagta gctactcaat aaatgcacat taccgctatc      60

gaaacggcca gcctgtgtgg caacttcctc acctcagtcc atctgacttc caacaggcac     120

ccgggagcac caagtaataa attgtgaaga gtacactttg aaacgtatca tgttctacgc     180

acatgtgtag ggtattatta tagttcaaga gaaagaggga acccgctggt gtctgagaag     240

aaacccgctg gtgtctgaga agaaacggtg aagggtgcac tacgaaggga atataaacta     300

atcaaacatt cttgaatggc aatgacagtc actaaagttg acacttcctg cagtctttta     360

gtttagaaac cacacttccc gagacgtccc ccatcccaac agggataaaa tcctctagct     420

caactcaggt atatccccac tattgggcca gggagacctg cggatggcga gagcaggttc     480

tcttaggagg gtgtggattg tgagcttgtg cgctggcgag gagcagaagg tcagggaggc     540

accgtagtta atgaatcaat aaaccaaaca gccgatcaat cacttcctgc agtccgagtc     600

cggcgagggc taatcgattg gtccgggtat tttgaaagga aaaacggcgg tggatatgtt     660

gtcaaacgga ggggtgcagg agaggcgggg ccaggcctcc aggtgacccc tctggcttcc     720

tgggcctgca gaggcgccga aacgtgcagg cgccggcgtc catgaggcgt ggggcgcgcc     780

gggcgagtcc ctgcttgcag gaggcgcccg agaggccgtc gcgtaccctg gcaagaggag     840

ctggttagca cctcccgacc ccgagaaggc ccagggggcg cccggcgggc ggggcgggga     900

ccgggcgagg cccgcctccc attggccgca tagcgccgcc cagcgcgggc cgccgttata     960

aagcagccgc cggcgccggg tgcctcacag cacgctgcca cgccgacgca gacccctctc    1020

tgcacgccag cccgcccgca cccaccatgg ccacagttca gcagctggaa ggaagatggc 1080

gcctggtgga cagcaaaggc tttgatgaat acatgaagga gctaggtgag gcacccggcc 1140

tggcagcgcc tgcaacgtgg cgtgttgtgc ggtcgtctgt ccctaggtcc ccgtcagcgt 1200

gccagattct ggggcaggag atgctcggcg gcctaccccc atcccctccc atcttcccca 1260

ccacgcggcc gttgggtgca gcgcgcgcag caccacgcgg gcaggcggcg aggagcaggg 1320

agcgtgcgcg cctcttgccc gcccgcgggc cgcaagattc cggaggtggt ccaccccgtg 1380

gtccacctac ctctgcttct ttcccttcgc ccaaacggca gcccttccgc attgcttcct 1440

gtcctttagc gcgcgcaccc gtcacctcac gctgcacttc tttcgacccc ctccaggcga 1500

ccctgtattt cccttttttc cccctttact catccttccc cttcctgcca ccattctgtc 1560

tctcccattt accccatcgt ggcagcaccc actccccttt ccctccctgt cgcatctttt 1620

gttccctgtg gcgcgcaggt caccctccgt tttcttcatg gggacgcggt gctggcgcgc 1680

agtttcccgc agaaatcctg tggaggggtc tgagtggcgc tacagcttca gcttgcattc 1740

ctctgtcagc cccgtctccc ccaggtcctc tgctcgccct taccagttga gccgaaccct 1800

ttggattggt accccatgga acaccagggc ccccgagaag cgtggcgctg gcggtgccga 1860

cctgctgctg gcactgccgg gccggggcgc cgcagtgggc gggtggcctg tgggaggagc 1920

gcaatgaggc ctgggggtgg ccgtgtgttg ccatcctggc ctctgccact tgagggtgag 1980

gaggaaggag gcagtgggct ttgctggaaa accgtaacag aaactgcctg gccctcctgc 2040

ggctaactgc atgcaagatg ggtgtggccc tgcagaaggc agatgacttc ttgaagcgtt 2100

ccgagggaga atgaatctca gtagtaagat tcatttctca ctcaaaacag tgcttcatta 2160

taaattactg tccttttcta tgccagtgac agattgcatg ctgattgaga cactggataa 2220

attgcaaaca aaaatggacc tttgtcacag gccactagga agtgagatgg agttagcata 2280

gcatggatcc tctctggaag ggagcttcca gccctaaggg atggctggga tttatgaggg 2340

tgcttcagaa gccggtatcc acaaatgtta acccggttgc tcgctggtga aataactacc 2400

gtgaccctct attgatacct ggtcgccgtg gcacttcgga atcactcctt cagtaccctt 2460

tatccaagac catgtggtgt taaggctggt ttatggtgaa tcatgagtgt atttttgata 2520

ctatcagtaa agaaggcagt atttttcttt ttccttgtaa acctacatca agatgactct 2580

ggaaaaagcg atttactgca cctgtccaat aaataacagt gtgtgtgtgt gtgtgtgtgt 2640

gtgtgtgtgt acaagtttaa ggcccaaaga acacttttct ctgcctgtag aaagcaaatt 2700

gtgtgtaggt ttggaggcct tgtttccagg attctggagt cttgaaggtt gtcaagactc 2760

agttcattag ggagcagtca caatgtgaaa agagcagagc ccatttattt gtccaaagtg 2820

agagtacata atgactcaat taacagcata aggcaccagc cctgagctgg caaaagagaa 2880

ggctccaagg ggaactttaa agcctggctg ggtgttcttc atcaagacaa tcatgctgcg 2940

taggttgtag tctcctcatt tatgaaacag tgatttggcc tggatctaag attccttata 3000

catgttaact aactctaagg ggaaagagat agatcataaa ttacatgtta acgttgaggg 3060

gaaattgata gatcataaat taaaatataa tttaatatgt tatatatttc tattgattta 3120

tatacctatg aaatagtttt tatattgaaa ggtaggattt tttttcccat ttgttagcat 3180

catgattgtg ctgctgagtt tttaaagatt gtgctatttg aatatctcag gatcaatttc 3240

tattttgtgt agtctattaa tcaaatattt atcgagaaca tttatatgcc agactgtgct 3300

ggagtcatag aatgtaatgg ttggcagaat ctagtaaaaa gaaaaagatt gagcaaactc 3360

ctgaatttgc aattttgaat gctttatctc caccttctat caactgcttt gagtaaataa 3420

aatttggaag tccacatttc ttccctaact ttctgaaagc cagcatagca tggtggccag 3480

agctcagatt tggagccagg gtgccagtgc tcttggttcc cagctccatc actttctagt 3540

tgaatgatct tgggcaagtt acttccgtgc ctcgctttct catcagtaaa atggagacag 3600

taacatctgc cttttacagg gctgttgtaa ggcttacctg aatttcatat atgtaaagtg 3660

ctggctcata ccgtgggata tatgtttaca atagttatca ataagcaaat aagtgaaaat 3720

atgccgcaca aagtgattac aatggaatta ttgcttttat catgtaaatg ataactttgg 3780

tcttcctgtt atttaacatg acttaacatt ctacaggagt gggaatagct ttgcgaaaaa 3840

tgggcgcaat ggccaagcca gattgtatca tcacttgtga tggtaaaaac ctcaccataa 3900

aaactgagag cactttgaaa acaacacagt tttcttgtac cctgggagag aagtttgaag 3960

aaaccacagc tgatggcaga aaaactcag 3989

<210> 2
<211> 20
<212> DNA
<213> Artificial sequence

<220>
<223> Primer

<400> 2
   tggtttttgg ggtttgtaga 20

<210> 3
<211> 22

<212>  DNA
<213>  Artificial sequence

<220>
<223>  Primer

<400>  3
atacctcacc taactccttc at                                                22


<210>  4
<211>  20
<212>  DNA
<213>  Artificial sequence

<220>
<223>  Primer

<400>  4
tttgggtttg tagaggcgtc                                                   20


<210>  5
<211>  20
<212>  DNA
<213>  Artificial sequence

<220>
<223>  Primer

<400>  5
ttaccaaaat acgcgacgac                                                   20



<210>  6
<211>  23
<212>  DNA
<213>  Artificial sequence

<220>
<223>  Primer

<400>  6
ttttttgggt ttgtagaggt gtt                                               23


<210>  7
<211>  23
<212>  DNA
<213>  Artificial sequence

<220>
<223>  Primer

<400>  7
accaaaatac acaacaacct ctc                                               23

<210> 8
<211> 20
<212> DNA
<213> Artificial sequence

<220>
<223> Primer

<400> 8
tcagcagctg gaaggaagat 20


<210> 9
<211> 20
<212> DNA
<213> Artificial sequence

<220>
<223> Primer

<400> 9
gccatcagct gtggtttctt 20